# EUROPEAN PATENT APPLICATION

(11) **EP 2 233 591 A1**
(43) Date of publication of application: **29.09.2010**
(21) Application number: 09156200.9
(22) Date of filing: 25.03.2009
(51) Int. Cl.: C12Q 1/70

(54) **Genotyping of bovine papillomavirus genotypes**

(71) Applicant: DKFZ Deutsches Krebsforschungszentrum, 69120 Heidelberg (DE)
(72) Inventor: Schmitt, Markus, 69121 Heidelberg (DE); Müller, Martin, 69151 Neckargemünd (DE)
(74) Representative: Stößel, Matthias

(57) **Abstract**

The present invention is concerned with the provision of diagnostic means and methods. Specifically, it relates to a composition comprising oligonucleotides selected from at least two different groups of oligonucleotides, said groups comprising at least one pair of oligonucleotides being capable of specifically amplifying polynucleotides comprised by a Bovine Papillomavirus (BPV), said BPV being selected from the group consisting of BPV-1, BPV-2, BPV-3, BPV-4, BPV-5, BPV-6, BPV-7, BPV-8, BPV-9, BPV-10, and BAPV-11 as well as uses based on said composition and kits comprising it. Moreover, contemplated is a method for the simultaneous detection and / or identification of BPV types in a sample.

SEQUENCE LISTING

<110>  DKFZ Deutsches Krebsforschungszentrum, Stiftung des
       öffentlichen Rechts

<120>  Genotyping of bovine papillomavirus genotypes

<130>  DK66189EP

<160>  30

<170>  PatentIn version 3.5

<210>  1
<211>  22
<212>  DNA
<213>  artificial

<220>
<223>  Fw1

<400>  1
tttataacag taggggacaa ta                                              22


<210>  2
<211>  25
<212>  DNA
<213>  artificial

<220>
<223>  Bw1


<220>
<221>  misc_feature
<222>  (11)..(11)
<223>  n = inosine

<400>  2
ccaaaataaa ngctagctta tattc                                           25


<210>  3
<211>  23
<212>  DNA
<213>  artificial

<220>
<223>  Fw2

<400>  3
tttttaactg taggggataa cac                                             23


<210>  4
<211>  25
<212>  DNA
<213>  artificial

<220>
<223>  Bw2

<400>  4
ccagaataac ggctagctta tattc                                           25


<210>  5
<211>  23
<212>  DNA

<213>  artificial

<220>
<223>  Fw3


<220>
<221>  misc_feature
<222>  (6)..(6)
<223>  n = inosine

<220>
<221>  misc_feature
<222>  (15)..(15)
<223>  n = inosine

<400>  5
tttgtnactg ctgtngacag tac                                              23


<210>  6
<211>  25
<212>  DNA
<213>  artificial

<220>
<223>  Bw3


<220>
<221>  misc_feature
<222>  (4)..(4)
<223>  n = inosine

<400>  6
gcanaattaa ggacaattcc cattc                                            25


<210>  7
<211>  23
<212>  DNA
<213>  artificial

<220>
<223>  Fw4

<400>  7
tttgttacag ctgttgatag tac                                              23


<210>  8
<211>  23
<212>  DNA
<213>  artificial

<220>
<223>  Fw5

<400>  8
tttgtgaccg ctgtggacag cac                                              23


<210>  9
<211>  25
<212>  DNA
<213>  artificial

<220>
<223>  Bw4

<400>  9
gcattataca tgatagttcc cactc                                          25


<210>  10
<211>  23
<212>  DNA
<213>  artificial

<220>
<223>  Fw6

<400>  10
tttgtgacaa ccatggataa ttc                                            23


<210>  11
<211>  25
<212>  DNA
<213>  artificial

<220>
<223>  Bw5

<400>  11
ctaaaacaaa catcagctca tagat                                          25


<210>  12
<211>  23
<212>  DNA
<213>  artificial

<220>
<223>  Fw7

<400>  12
tttataacgg taggtgacaa tag                                            23


<210>  13
<211>  25
<212>  DNA
<213>  artificial

<220>
<223>  Bw6

<400>  13
ctagtatgac agccagctta tattc                                          25


<210>  14
<211>  23
<212>  DNA
<213>  artificial

<220>
<223>  Fw8

<400>  14
tttgtcactg ctgtggacag tac                                            23


<210>  15
<211>  25
<212>  DNA
<213>  artificial

<220>

<223>  Bw7

<400>  15
gaagtataag tgaaagttcc cattc                                          25


<210>  16
<211>  23
<212>  DNA
<213>  artificial

<220>
<223>  Fw9

<400>  16
ttcataacag cagtagatac tac                                            23


<210>  17
<211>  25
<212>  DNA
<213>  artificial

<220>
<223>  Bw8

<400>  17
gcatgattag tgagacctcc cactc                                          25


<210>  18
<211>  23
<212>  DNA
<213>  artificial

<220>
<223>  Fw10

<400>  18
tttgttacag tcatggacaa ctc                                            23


<210>  19
<211>  25
<212>  DNA
<213>  artificial

<220>
<223>  Bw9

<400>  19
tcaaaatgaa ggaaagctca tatat                                          25


<210>  20
<211>  19
<212>  DNA
<213>  artificial

<220>
<223>  probe BPV 1

<400>  20
atggaacccc actaacaga                                                 19


<210>  21
<211>  19
<212>  DNA
<213>  artificial

<220>
<223>  probe BPV2

<400>  21
atggaaacgc attgtcaga                                                    19


<210>  22
<211>  21
<212>  DNA
<213>  artificial

<220>
<223>  probe BPV 3

<400>  22
tttagaggac caggatactt a                                                 21


<210>  23
<211>  21
<212>  DNA
<213>  artificial

<220>
<223>  probe BPV 4

<400>  23
taaaccacaa gaaacttata c                                                 21


<210>  24
<211>  19
<212>  DNA
<213>  artificial

<220>
<223>  BPV 5

<400>  24
acgataggaa acctgagca                                                    19


<210>  25
<211>  20
<212>  DNA
<213>  artificial

<220>
<223>  probe BPV 6

<400>  25
acctcaggac ctgtacaaat                                                   20


<210>  26
<211>  20
<212>  DNA
<213>  artificial

<220>
<223>  probe BPV 7

<400>  26
tcctaatccc actgctcaat                                                   20


<210>  27
<211>  19

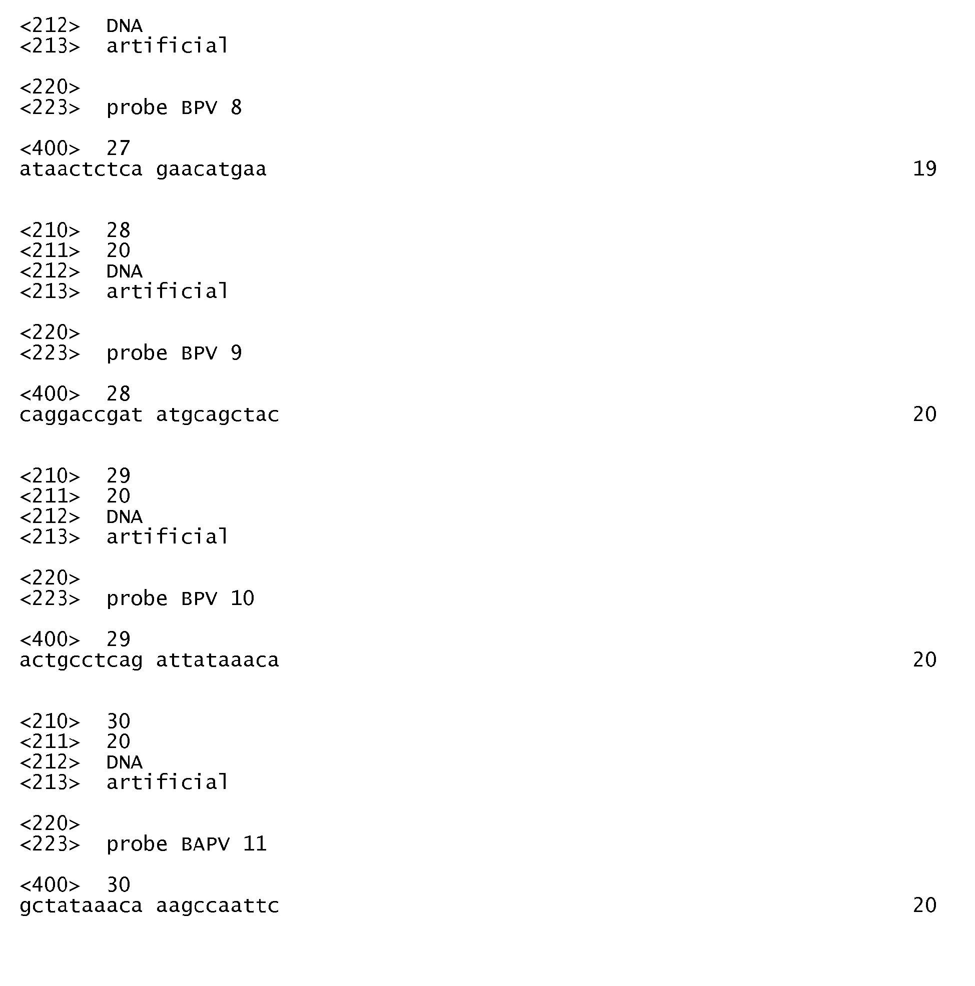

## Description

The present invention is concerned with the provision of diagnostic means and methods. Specifically, it relates to a composition comprising oligonucleotides selected from at least two different groups of oligonucleotides, said groups comprising at least one pair of oligonucleotides being capable of specifically amplifying polynucleotides comprised by a Bovine Papillomavirus (BPV), said BPV being selected from the group consisting of BPV-1, BPV-2, BPV-3, BPV-4, BPV-5, BPV-6, BPV-7, BPV-8, BPV-9, BPV-10, and Bovine alimentary Papilloma Virus-11 (BAPV-11) as well as uses based on said composition and kits comprising it. Moreover, contemplated is a method for the simultaneous detection and / or identification of BPV types in a sample.

Bovine Papillomaviruses (BPV), including Bovine alimentary Papilloma Virus-11 (BAPV-11), are a group of DNA viruses from the family of Papillomaviridae which are associated with several forms of cutaneous and mucosal papilloma in cattle. Based on sequence relatedness, eleven different types have been characterized so far. Infections caused by BPV are common in cattle, with around 50% of cattle being estimated to bear lesions (warts) in the UK (Campo MS (1995) Infection by bovine papillomavirus and prospects for vaccination. Trends Microbiol. 3:92-97).

Warts arising in cutaneous regions of cattle mostly are non-problematic and can regress spontaneously due to the immune response of the animal. They are, however, economically damaging to the owners of show cattle, since affected animals are not admitted to trade shows due to the high contagiousity of the infection. More economic damage is caused by warts of the genital areas, since they lead to a decrease or even the loss of reproductive functions both in male and in female cattle. Warts on teats may cause mastitis and interfere with suckling and milking.

Apart from infecting cattle, BPV-1 and -2 have been implicated in the genesis of equine sarcoid, an ulceration of the horse skin (L Nasir et al. (2008) Bovine papillomaviruses: their role in the aetiology of cutaneous tumours of bovids and equids. Vet Dermatol. 19(5):243-54).

Methods of managing the spread of BPV infections in droves mainly rely on activating the animals' immune response, i.e. vaccination. One problem with prophylactic vaccination of cattle with BPV-derived antigens is that protection usually is type-specific, i.e. protection is only conferred against the BPV type the antigen was derived from. It would therefore be highly advantageous to have means and methods that enable the veterinarian to monitor the efficacy of future BPV vaccines, to identify novel vaccine candidates, and to identify the type(s) of BPV involved in an infection in order to make it possible to vaccinate other members of the drove using the correct vaccine.

Attempts to identify BPV types in samples have been made. So far, polymerase chain reaction (PCR) detection systems derived from human detection systems have been used (Antonsson and Hansson (2002) Healthy skin of many animal species harbors papillomaviruses which are closely related to their human counterparts. J Virol 76(24):12537-12542; Ogawa et al. (2004) Broad-spectrum detection of papillomaviruses in bovine teat papillomas and healthy teat skin. J Gen Virol 85:2191-2197) to detect and identify BPV types. However, since these systems were originally designed to detect human papillomaviruses and due to sequence variations between human and bovine papillomaviruses, the sensitivity of the PCR systems employed is low. Especially, BPV-2, BPV-4, BPV-7, and BPV-8 have not yet been detected with these systems. Furthermore, these methods rely on gel electrophoresis of PCR-products followed by sequencing in order to identify BPV types, which is a time- and cost-intensive procedure. Moreover, in the case of multiple infections, only one BPV can be identified in one reaction container, since only the BPV Type with the highest viral load will generate sufficient signals in the sequencing reaction.

Thus, the technical problem underlying the present invention may be seen as the provision of means and methods for reliably detecting and identifying BPV present in a sample without the drawbacks as referred to above. The technical problem is solved by the embodiments characterized in the claims and herein below.

Accordingly, the present invention relates to a composition comprising oligonucleotides selected from at least two different groups of oligonucleotides, said groups comprising at least one pair of oligonucleotides being capable of specifically amplifying polynucleotides comprised by a Bovine Papillomavirus (BPV), said BPV being selected from the group consisting of BPV-1, BPV-2, BPV-3, BPV-4, BPV-5, BPV-6, BPV-7, BPV-8, BPV-9, BPV-10, and BAPV-11.

The term "composition comprising oligonucleotides" as meant herein relates to a mixture of different oligonucleotide molecular species, preferably of oligonucleotide groups as specified elsewhere in this application. In addition, a composition may comprise further components other than the oligonucleotides, e.g. components for the amplification of polynucleotides, preferably by PCR. Such components may be, but are not limited to, an aqueous buffer, a water soluble magnesium salt, deoxythymidine triphosphate (dTTP), deoxyadenosine triphosphate (dATP), deoxycytidine triphosphate (dCTP), and deoxyguanosine triphosphate (dGTP), and a DNA polymerase, e.g. one of the thermostable DNA polymerases from Thermus aquaticus or from Pyrococcos furiosus.

The term "group of oligonucleotides" as used herein, preferably, relates to a composition comprising a plurality of oligonucleotide pairs. Preferably, an oligonucleotide group is specific for one type of BPV.

The term "pair of oligonucleotides" relates to two oligonucleotides capable of amplifying polynucleotides that are specific for one type of BPV. The sequences of the preferred corresponding pairs of oligonucleotides as well as the corresponding targets and probe oligonucleotides are shown in table 1.

The term "oligonucleotide" as used herein relates to a composition comprising oligonucleotide molecular species wherein 80%, 85%, 90%, 95%, 99%, all molecules of the molecular species have a specific nucleic acid sequence. Preferably, the term "oligonucleotide" relates to a primer for DNA amplification techniques such as PCR (whereas the term "probe oligonucleotides", preferably, relates to probes, see herein below). An oligonucleotide shall comprise a number of nucleotides being sufficient for specific binding to a sequence stretch of a target polynucleotide. Preferably, an oligonucleotide as meant herein has between 15 and 30 nucleotides in length, more preferably between 18 and 28 nucleotides in length, and most preferably between 23 and 25 nucleotides in length. Preferably, the sequence of the oligonucleotide is not degenerated and, preferably, the oligonucleotide does not contain base analogues other than inosine. Preferably, the oligonucleotide is a single-stranded oligodesoxyribonucleotide. However, due to self-complementarity, the oligonucleotide may be partially double-stranded under certain conditions (depending on, e.g., the sequence of the oligonucleotide, the salt concentration, and the temperature). Particularly preferred oligonucleotides have the specific sequences and / or properties referred to herein.

The oligonucleotides of the present invention, preferably, are used as starting molecules for the synthesis of a polynucleotide which is sufficiently complementary to the nucleic acid strand to be copied by an appropriate amplification technique for polynucleotides, preferably by PCR.

The amount and / or the concentration of the oligonucleotides may be any amount or concentration deemed appropriate. The amounts and / or concentrations of the individual oligonucleotides of the invention may differ and, thus, a solution comprising said oligonucleotides does not have to be equimolar with respect to the oligonucleotides. A preferred concentration of each oligonucleotide is between 0.5 and 10 µM, more preferable 1 and 5 µM, most preferable 2 and 4 µM. Moreover, it is to be understood that the person skilled in the art is able to adjust the concentrations of the oligonucleotides of the invention in order to optimize the amplification of specific polynucleotides as referred to herein and, thus, to optimize the detection of BPV in a sample without further ado.

The oligonucleotides of the present invention may be labelled or contain other modifications which allow a detection and / or analysis of an amplification product and / or the binding to a carrier. Labelling can be done by various techniques well known in the art and depending on the label to be used. Particularly, the oligonucleotides may be biotinylated in order to enable the binding of the amplification products to a streptavidin surface or fluorescent conjugate. Moreover, labels to be used in the context of the present invention may be, but are not limited to, fluorescent labels comprising, inter alia, fluorochromes such as R-phycoerythrin, Cy3, Cy5, fluorescein, rhodamin, Alexa, or Texas Red. However, the label may also be an enzyme or an antibody. It is envisaged that an enzyme to be used as a label will generate a detectable signal by reacting with a substrate. Suitable enzymes, substrates and techniques are well known in the art. An antibody to be used as a label may specifically recognize a target molecule which can be detected directly (e.g., a target molecule which is itself fluorescent) or indirectly (e.g., a target molecule which generates a detectable signal, such as an enzyme). The oligonucleotides of the present invention may also contain 5' restriction sites, locked nucleic acid molecules (LNA) or be part of a peptide nucleotide acid molecule (PNA). Such PNA can, in principle, be detected via the peptide part by, e.g. antibodies.

The term "specifically amplifying", preferably, relates to amplifying a nucleic acid comprised in a BPV and confined by a pair of oligonucleotides without amplifying other nucleic acids, said polynucleic acid comprising polynucleotide molecular species wherein all, 99%, 95%, 90%, 80%, 70%, 60% of molecules of the molecular species have a specific nucleic acid sequence.

The term "BPV" as used herein relates to viruses from the family *Papillomaviridae* that infect cattle. "BPV type" relates to a subgroup of BPV distinguished on the basis of sequence relatedness. BPV types known are BPV-1 (GenBank Accession No: X02346.1, GI:60965), BPV-2 (GenBank Accession No: M20219.1, GI:332996), BPV-3 (GenBank Accession No: AF486184.1, GI:22901309), BPV-4(GenBank Accession No: X05817.1, GI:60862), BPV-5 (GenBank Accession No: AF457465.1, GI:22901263), BPV-6 (GenBank Accession No: AJ620208.1, GI:40804501), BPV-7 (GenBank Accession No: DQ217793.1, GI:78057396), BPV-8 (GenBank Accession No: DQ098913.1, GI:89034256), BPV-9 (GenBank Accession No: AB331650.1, GI:163914074), and BPV-10 (GenBank: Accession No: AB331651.1, GI:163914081), and BAPV-11 (partial sequence GenBank Accession No: AY300820.1, GI:34537330).

The aforementioned composition is herein also referred to as "composition comprising oligonucleotides".

Advantageously, it was shown in the studies underlying the present invention that determining BPV types in a sample comprising the steps of contacting a sample with a composition comprising oligonucleotides as referred to herein and detecting and / or identifying BPV types based on the amplified polynucleotides by applying said compositions is required for sufficient detection and / or identification of BPV types in a sample. The compositions referred to herein allow for a highly reliable detection and / or identification of BPV types simultaneously, in a cost- and time-effective manner.

The definitions made above apply *mutatis mutandis* to the following:

Moreover, in a preferred embodiment of the composition of the present invention, said composition comprises oligonucleotides, wherein said at least two groups of oligonucleotides are selected from the group consisting of
a) a group of oligonucleotides comprising
   i) a pair of oligonucleotides having a nucleic acid sequence as shown in SEQ ID NO: 1 and SEQ ID NO: 2 or ii) at least one pair of oligonucleotides which are capable of specifically amplifying polynucleotides which are specifically amplified by the oligonucleotides i); and
b) a group of oligonucleotides comprising
   i) a pair of oligonucleotides having a nucleic acid sequence as shown in SEQ ID NO: 3 and SEQ ID NO: 4 or
   ii) at least one pair of oligonucleotides which are capable of specifically amplifying polynucleotides which are specifically amplified by the oligonucleotides i); and
c) a group of oligonucleotides comprising
   i) a pair of oligonucleotides having a nucleic acid sequence as shown in SEQ ID NO: 5 and SEQ ID NO: 6 or
   ii) at least one pair of oligonucleotides which are capable of specifically amplifying polynucleotides which are specifically amplified by the oligonucleotides i); and
d) a group of oligonucleotides comprising
   i) a pair of oligonucleotides having a nucleic acid sequence as shown in SEQ ID NO: 7 and SEQ ID NO: 6 or
   ii) at least one pair of oligonucleotides which are capable of specifically amplifying polynucleotides which are specifically amplified by the oligonucleotides i); and
e) a group of oligonucleotides comprising
   i) a pair of oligonucleotides having a nucleic acid sequence as shown in SEQ ID NO: 8 and SEQ ID NO: 9 or
   ii) at least one pair of oligonucleotides which are capable of specifically amplifying polynucleotides which are specifically amplified by the oligonucleotides i); and
f) a group of oligonucleotides comprising
   i) a pair of oligonucleotides having a nucleic acid sequence as shown in SEQ ID NO: 10 and SEQ ID NO: 11 or
   ii) at least one pair of oligonucleotides which are capable of specifically amplifying polynucleotides which are specifically amplified by the oligonucleotides i); and
g) a group of oligonucleotides comprising
   i) a pair of oligonucleotides having a nucleic acid sequence as shown in SEQ ID NO: 12 and SEQ ID NO: 13 or
   ii) at least one pair of oligonucleotides which are capable of specifically amplifying polynucleotides which are specifically amplified by the oligonucleotides i); and
h) a group of oligonucleotides comprising
   i) a pair of oligonucleotides having a nucleic acid sequence as shown in SEQ ID NO: 14 and SEQ ID NO: 15 or
   ii) at least one pair of oligonucleotides which are capable of specifically amplifying polynucleotides which are specifically amplified by the oligonucleotides i); and
i) a group of oligonucleotides comprising
   i) a pair of oligonucleotides having a nucleic acid sequence as shown in SEQ ID NO: 16 and SEQ ID NO: 17 or
   ii) at least one pair of oligonucleotides which are capable of specifically amplifying polynucleotides which are specifically amplified by the oligonucleotides i); and j) a group of oligonucleotides comprising
   i) a pair of oligonucleotides having a nucleic acid sequence as shown in SEQ ID NO: 18 and SEQ ID NO: 19 or
   ii) at least one pair of oligonucleotides which are capable of specifically amplifying polynucleotides which are specifically amplified by the oligonucleotides i).

Moreover, said composition may comprise oligonucleotides selected from at least three, four, five, six, seven, eight, nine or all of the aforementioned groups of oligonucleotides.

Furthermore, the present invention also relates to a composition comprising at least two, three, four, five, six, seven, eight, nine, or ten probe oligonucleotides, said oligonucleotides being capable of specifically hybridizing to polynucleotides comprised by a BPV, said BPV being selected from the group consisting of BPV-1, BPV-2, BPV-3, BPV-4, BPV-5, BPV-6, BPV-7, BPV-8, BPV-9, BPV-10, and BPV-11.

A "probe oligonucleotide" in the context of the present invention, preferably, is a single-stranded nucleic acid molecule that can specifically hybridize with a polynucleotide that can be amplified by using the oligonucleotides according to the invention. A probe oligonucleotide comprises a stretch of nucleotides that specifically hybridize with the target sequence.

Thus, it is at least partially complementary to the target polynucleotide. Said stretch of nucleotides is, preferably, 85%, 90%, 95%, 99% or more preferably 100% identical to the complementary strand of a sequence region comprised by a target polynucleotide. Preferably, a probe oligonucleotide as meant herein is between 15 and 30 nucleotides in length, and most preferably between 18 and 23 nucleotides in length. Preferably, the probe oligonucleotides are bound to a carrier providing a solid surface. More preferably, said carrier is a small particle or bead. The overall size of a small particle or bead, preferably, may be in the micrometer or nanometer range. Preferably, said beads and particles may be stained with a specific dye, more preferably with a specific fluorescent dye. Preferably, by staining various carriers with various dyes, the carriers can be distinguished from each other. By using a carrier with a specific dye for a specific probe oligonucleotide (thus, a nucleic acid that targets the amplified polynucleotides of a specific BPV type), said carrier is distinguishable from other carriers comprising different dyes. In one preferred embodiment, commercially available Luminex microspheres (Luminex Corp., Austin, Texas, USA) are used. Thus, for determining BPV types the BPV type-specific probes are coupled to fluorescence-labelled polystyrene beads (Luminex suspension array technology) which are hybridized with the amplification products under suitable, preferably, stringent conditions. Moreover, the amplification products may be identified by use of microarrays, Reverse-Line blots (RLB), Dot blots or similar technologies which contain BPV type specific oligonucleotides linked to a suitable carrier.

Preferably, the probe oligonucleotides that specifically bind to the amplified polynucleotides are selected from the group consisting of probe oligonucleotides comprising a nucleic acid sequence as shown in SEQ ID NO: 20 (allows the detection of the BPV-1 L1 gene and, thus, of BPV-1), SEQ ID NO: 21 (allows the detection of the BPV-2 L1 gene and, thus, of BPV-2), SEQ ID NO: 22 (allows the detection of the BPV-3 L1 gene and, thus, of BPV-3), SEQ ID NO: 23 (allows the detection of the BPV-4 L1 gene and, thus, of BPV-4), SEQ ID NO: 24 (allows the detection of the BPV-5 L1 gene and, thus, of BPV-5), SEQ ID NO: 25 (allows the detection of the BPV-6 L1 gene and, thus, of BPV-6), SEQ ID NO: 26 (allows the detection of the BPV-7 L1 gene and, thus, of BPV-7), SEQ ID NO: 27, allows the detection of the BPV-8 L1 gene and, thus, of BPV-8), SEQ ID NO: 28 (allows the detection of the BPV-9 L1 gene and, thus, of BPV-9), SEQ ID NO: 29 (allows the detection of the BPV-10 L1 gene and, thus, of BPV-10), SEQ ID NO: 30 (allows the detection of the BAPV-11 L1 gene and, thus, of BAPV-11).

The nucleic acid sequences of the probe oligonucleotides as well as the BPV types that are detected and / or identified using said probe oligonucleotides are listed in table 1. Also shown in table 1 are the corresponding amplified polynucleotides as well as the oligonucleotide pairs that amplify these polynucleotides.

In another preferred embodiment of the composition of the present invention, the probe oligonucleotides comprised by the composition are capable of specifically hybridizing to polynucleotides which are amplified by the oligonucleotides as referred to above. More preferably, said probe oligonucleotides have a nucleic acid sequence as shown in SEQ ID NOs: 20 to 31 or said probe oligonucleotides are capable to specifically hybridize to the antisense strand of oligonucleotides that have nucleic acid sequences as shown in SEQ ID NOs: 20 to 31.

The present invention relates to a method for the simultaneous detection and / or identification of BPV types in a sample comprising the steps of
a) contacting a sample with a composition containing oligonucleotides as referred to above under conditions which allow for the amplification of polynucleotides and
b) determining the presence and / or identity of BPV in said sample based on the amplified polynucleotides obtained in step a).

The method of the present invention, preferably, is an *in vitro* method. Moreover, it may comprise steps in addition to those explicitly mentioned above. For example, further steps may relate to sample pre-treatments or evaluation of the results obtained by the method. Additionally, internal controls, such as DNA quality controls or PCR performance controls may be used. The method of the present invention may be also useful for confirmation of BPV types present in a sample. The method may be carried out manually or assisted by automation. Preferably, step (a) and / or (b) may in total or in part be assisted by automation, e.g. by suitable robotic and sensory equipment for the determination in step (b).

In the term "simultaneous detection" as used herein, "detection" relates to assessing the presence or absence of BPV types in a sample. The detection of BPV types is accomplished by detecting amplified polynucleotides that are specific for the BPV types to be detected. "Simultaneous detection" relates to amplifying polynucleotides that are specific for at least two, three, four, five, six, seven, eight, nine, ten, eleven types of BPV in one container, thus, in the same container. A preferred container is a reaction tube. Such reaction tubes are well known in the art and, e.g., are commonly referred to as PCR tubes. It is to be understood that the form of these tubes may depend on the PCR apparatus used for the amplification of BPV-specific polynucleotides.

The term "identification of BPV types", preferably, relates to assessing the presence or absence of one specific type of the various, individual BPV types. Preferably, two, three, four, five, six, seven, eight, nine, ten, eleven BPV types are identified simultaneously. The identification of BPV types is accomplished by identifying amplified polynucleotides that are specific for the respective BPV type to be identified. Preferably, the amplification of polynucleotides that are specific for BPV types is done in only one container, thus, the same container. A preferred container has been described herein before.

The identification of the BPV types is based on the amplified polynucleotides, and relates to identifying amplified polynucleotides that are specific for the various, individual BPV types. Preferably, this will be achieved by detecting the presence or absence of amplified polynucleotides that are specific for the BPV type to be identified. If an amplified polynucleotide that is specific for an individual BPV type is present, then the respective BPV type is present in the sample. If there is no amplification product for a polynucleotide that is specific for a certain BPV type and, thus, if an amplified polynucleotide is absent, the respective BPV type is not present in the tested sample. The identification of a plurality of amplified polynucleotides and, thus, of a plurality of BPV types can be done because of sequence variation between the amplified polynucleotides. Identifying the BPV types based on the amplified polynucleotides is, preferably, done by using suitable probe oligonucleotides. Thus, the identification of the various amplified polynucleotides, preferably, comprises the step of hybridizing the amplified polynucleotides with probe oligonucleotides that specifically bind to the amplified polynucleotides.

However, it is also contemplated by the present invention that the amplification products are identified by other means known in the art. The identification may be done by sequencing amplification product(s) and / or by restriction fragment length polymorphism (RFLP) analysis. The identification of BPV types can also be done by conducting microarray assays, southern blot analysis, dot blot assays, or by membrane-based reverse line blot.

It is also contemplated by the present invention that the efficacy of the detection is ensured by the inclusion of one or more positive controls (e.g. using plasmid DNA with cloned BPV-genomes and / or and bovine warts), preferably in a separate container.

The term "sample", preferably, relates to a specimen taken from a part of the body of an animal. Preferably, said animal is a mammal and most preferably said animal is a bovidae or an equidae. Said part of the body of an animal, preferably, is skin or mucosa and most preferably is skin or mucosa having developed warts, sarcoid lesions or other lesions suspected to be related to BPV infection. The methods for obtaining a specimen from the body of an animal are well known in the art. Preferably, specimens are obtained by removing small amounts of cells from the body, most preferably by excision or by taking a smear or a swab or plugging hairs. Preferably, the sample may have any volume and concentration deemed appropriate. Moreover the sample may have been further processed in order to carry out the method of the present invention. Particularly, polynucleotides such as DNA or RNA, preferably DNA, might be extracted and / or purified from the sample by methods and means known in the art (e.g. see Examples). Thus, the term sample may also relate to polynucleotides, preferably DNA, purified and /or extracted from any sample as mentioned above.

However, it is also contemplated by the present invention that the sample may be a cell culture sample, relating to a sample from cells which are maintained in vitro in a suitable cultivation medium. Preferably, said cells are eukaryotic cells, more preferably mammalian cells and most preferably bovine or equine cells. The sample may be or be derived from cells or from cell culture supernatant which may have been processed as referred to above. Furthermore, the sample may also be a liquid, a hair or a swab from a site suspected to comprise BPV.

The sample may contain more than one BPV type, i.e. a plurality of BPV types. Preferably, a sample comprises at least one BPV type. At least one means one or more than one, at least two, at least three, at least four, at least five, at least six, at least seven, at least eight, at least nine, at least ten, or eleven BPV types. However, a sample suspected to comprise BPV may also turn out to be free of BPV, i.e. does not comprise any BPV that can be detected by applying the method of the present invention.

The term "contacting" as used in the context of the method of the present invention is understood by the skilled person. Preferably, the term relates to bringing a composition of the present invention into physical contact with a sample, e.g. by mixing the sample and the composition and thereby allowing the sample and the composition to interact.

The term "under conditions which allow for amplification of polynucleotides" as used herein is understood by the skilled person. The term relates to a template-dependent process which results in an increase of the amount of a nucleic acid molecule relative to its initial amount. In accordance with the present invention the amplification of a polynucleotide of interest shall allow its detection by any method deemed appropriate and / or, e.g., described herein below. The amplification of a polynucleotide of interest may be carried out by well-known methods, preferably by PCR, but also by reverse transcriptase PCR, real-time PCR, reverse transcriptase real-time PCR, Templex-PCR, nucleic-acid sequence based amplification (NASBA), and isothermal amplification methods using polymerases and specific oligonucleotides as primers. The aforementioned amplification methods are well known in the art. Preferred embodiments of a PCR in the context of the present invention are described in the Examples.

Thus, in another preferred embodiment of the present invention, the amplified polynucleotides of step a) in the method as referred to above are obtained by PCR.

In yet another preferred embodiment, the determination of the various amplified polynucleotides as in step b) in the method as referred to above comprises the step of hybridizing the amplified polynucleotides with probe oligonucleotides as referred to above.

Furthermore, the present invention relates to the use of a composition of the present invention (thus, of a composition comprising oligonucleotides and / or a composition comprising probe oligonucleotides as specified above) for detecting and / or identifying BPV types in a sample. Preferably, said BPV types are detected simultaneously.

Finally, the present invention relates to a kit, preferably adapted for carrying out the method of the present invention, comprising a composition comprising oligonucleotides and / or a composition comprising probe oligonucleotides as specified herein and an instruction manual.

The term "kit" as used herein refers to a collection of the aforementioned means, e.g., means for contacting a sample under conditions which allow for amplification of polynucleotides and for detection and / or identification of BPV types based on the amplified polynucleotides, preferably, provided separately or within a single container. The container, preferably, comprises instructions for carrying out the method of the present invention. The components of the kit are provided, preferably, in a "ready-to-use" manner, with concentrations adjusted accordingly.

All references cited in this specification are herewith incorporated by reference with respect to their entire disclosure content and the disclosure content specifically mentioned in this specification.

The following examples shall merely illustrate the invention. They shall not be construed, whatsoever, to limit the scope of the invention.

### Example 1

### BPV sequence alignment, BPV primer and probe design

The nucleotide sequences of the L1 regions of 11 BPV genotypes, i.e. BPV types 1, 2, 3, 4, 5, 6, 7, 8, 9 and 10, and BAPV-11, were obtained from the National Center for Biotechnology Information (NCBI) nucleotide sequence database (GenBank) and were aligned with T-COFFEE (Notredame, C., D. G. Higgins, and J. Heringa. 2000. T-Coffee: A novel method for fast and accurate multiple sequence alignment. J Mol Biol 302:205-17). The criteria for BSGP5+/6+ primer design were as follows: Pools of oligonucleotides rather than the addition of degenerate base sites in the primer sequences were preferred to avoid synthesis variation in the primer sequence. Any BPV type should have no more than two mismatches to at least one primer. Backward primers were biotinylated at the 5' terminus for labelling the target strand of the amplified product.

All novel probes were designed with no mismatch to the targeted BPV prototype sequence and all other variant sequences of this type available in the NCBI nucleotide sequence database (GenBank). In addition, each probe exhibited at least three mismatches with all other BPV type sequences.

Table 1 lists names, orientations (forward or backward) and nucleic acid sequences of probe and primer oligonucleotides having a nucleic acid sequence as shown in SEQ ID NOs: 1 to 30.

**Table 1. Probe and primer oligonucleotides overview**

| **BPV type** | **Probe oligonucleotide sequence 5'to 3'** | **Probe SEQ ID NO:** | **Primer name (F, forward; b, backward)** | **Primer sequence 5'to 3'** | **Primer SEQ ID NO:** |
|---|---|---|---|---|---|
| BPV 1 | ATGGAACCCCACTAACAGA | 20 | Fw1 | TTTATAACAGTAGGGGACAATA | 1 |
| | | | Bw1 | ccaaaataaaigctagcttatattc | 2 |
| BPV 2 | ATGGAAACGCATTGTCAGA | 21 | Fw2 | TTTTTAACTGTAGGGGATAACAC | 3 |
| | | | Bw2 | Ccagaataacggctagcttatattc | 4 |
| BPV 3 | TTTAGAGGACCAGGATACTTA | 22 | Fw3 | TTTGTiACtGCTGTiGACAGtAC | 5 |
| | | | Bw3 | gcaiaattaaggacaattcccattc | 6 |
| BPV 4 | TAAACCACAAGAAACTTATAC | 23 | Fw4 | TTTGTTACAGCTGTTGATAGTAC | 7 |
| | | | Bw3 | gcaiaattaaggacaattcccattc | 6 |
| BPV 5 | ACGATAGGAAACCTGAGCA | 24 | Fw1 | TTTATAACAGTAGGGGACAATA | 1 |
| | | | Bw1 | ccaaaataaaigctagcttatattc | 2 |
| BPV 6 | ACCTCAGGACCTGTACAAAT | 25 | Fw5 | TTTGTGACCGCTGTGGACAGCAC | 8 |
| | | | Bw4 | gcattatacatgatagttcccactc | 9 |
| BPV 7 | TCCTAATCCCACTGCTCAAT | 26 | Fw6 | TTTGTGACAACCATGGATAATTC | 10 |
| | | | Bw5 | ctaaaacaaacatcagctcatagat | 11 |
| BPV 8 | ATAACTCTCAGAACATGAA | 27 | Fw7 | TTTATAACGGTAGGTGACAATAG | 12 |
| | | | Bw6 | ctagtatgacagccagcttatattc | 13 |
| BPV 9 | CAGGACCGATATGCAGCTAC | 28 | Fw8 | TTTGTCACTGCTGTGGACAGTAC | 14 |
| | | | Bw7 | gaagtataagtgaaagttcccattc | 15 |
| BPV 10 | ACTGCCTCAGATTATAAACA | 29 | Fw9 | TTCATAACAGCAGTAGATACTAC | 16 |
| | | | Bw8 | gcatgattagtgagacctcccactc | 17 |
| BAPV11 | GCTATAAACAAAGCCAATTC | 30 | Fw10 | TTTGTTACAGTCATGGACAACTC | 18 |
| | | | Bw9 | tcaaaatgaaggaaagctcatatat | 19 |

### Example 2

### Development of the BPV PCR

For BPV amplification, 2 µL of DNA extracted from cattle warts or hairs or 1 µL of BPV plasmid dilutions were amplified in 50 µL containing 1 x QIAGEN Multiplex PCR Master Mix (3 mM MgCl₂, dNTP mix, 0.5 x Q-solution and HotStarTaq DNA polymerase), and 0.2 µM each primer. Using a Mastercycler (Eppendorf, Germany) PCR products were amplified by incubation at 95°C for 15 minutes to activate the enzyme, followed by 40 cycles of denaturation at 94°C for 30 seconds, annealing at 38°C for 90 seconds, and extension at 71 °C for 80 seconds. Final extension was performed at 72°C for 4 minutes and reactions were stored at 4°C.

### Example 3:

### Coupling of oligonucleotide probes.

The sequences of 5'amino-modified C-12-linked oligonucleotide probes (purchased from MWG-Biotech) are shown in Table 1. Probes were coupled to carboxylated beads by a carbodiimide-based coupling procedure. For each combination of probe and bead set, 2.5 million carboxylated beads (xMAP; Luminex Corp., Austin, TX) were suspended in 25 µl of 0.1 M 2-(N-morpholino)ethanesulfonic acid, pH 4.5 (MES). Probe oligonucleotides (400 pmol) and 200 µg of N-(3-dimethylaminopropyl)-N-ethylcarbodiimide (EDC) were added and thoroughly mixed with the beads. Incubation was performed in the dark under agitation for 30 min and was interrupted by a thorough mixing step after 15 min. The addition of EDC and incubation steps were repeated and the coupled beads were finally washed once with 0.5 ml of 0.2 g/liter Tween-20 and once with 0.5 ml of 1.0 g/liter sodium dodecyl sulfate before being stored in 100 µl of TE buffer at 4°C in the dark.

### Example 4:

### Development of BPV hybridization method.

PCR products were denatured and hybridized to bead-coupled BPV-specific oligonucleotide probes in 96-well plates, allowing PCR products from 96 specimens to be processed in parallel.

Following multiplex PCR amplification, 10 µl of each reaction mixture were transferred to 96-well plates containing 33 µl of tetramethylammonium chloride (TMAC) hybridization solution (0.15 M TMAC, 75 mM Tris-HCl, 6 mM EDTA, 1.5 g/liter Sarkosyl, pH 8.0), 7.0 µl of 1 x TE, and a mixture of 2,000 probe-coupled beads of each set. The mixture was heated to 95°C for 10 min in a laboratory oven, immediately placed on ice for 1 min, and then transferred to a thermomixer. Hybridization was performed at 41°C for 30 min under agitation. The samples were transferred to a 96-well wash plate (Millipore, Bedford, MA), pre-equilibrated with washing buffer (phosphate-buffered saline, 0.02% Tween). Subsequently, the beads were washed once with 100 µl of washing buffer on a vacuum wash station (Millipore). On a horizontal shaker at room temperature, beads were resuspended for 20 min in 50 µl of streptavidin-R-phycoerythrin (Strep-PE; Molecular Probes, Eugene, OR) diluted 1:1,600 in 2.0 M TMAC, 75 mM Tris-HCl, 6 mM EDTA, 1.5 g/liter Sarkosyl, pH 8.0. Beads were then washed three times with 100 µl washing buffer and finally resuspended in 100 µl washing buffer for 5 min on a shaker. Beads were analyzed for internal bead color and R-phycoerythrin reporter fluorescence on a Luminex 100 analyzer. The median reporter fluorescence intensity (MFI) of at least 100 beads was computed for each bead set in the sample.

In order to determine the specificity of the hybridization, BPV-specific probes were coupled individually to defined bead sets and hybridized to 10 µl (100 to 300 ng DNA) of PCR products derived from PCRs on bacterial colonies containing the BPV genome of interest. Detection of all BPV types was highly specific and no cross-hybridization was observed (data not shown).

### Example 5

### Analytic sensitivity.

The analytic sensitivity of the BPV primers was determined using 10-fold dilutions series of plasmids containing genomic DNA from BPV types 1, 2, 3, 4, 5 and 6 in the presence of 100 ng of human placenta (HP-) DNA. PCR products were analyzed by the Luminex-based Multiplex BPV Genotyping assay. The BPV primer set detected all BPV genotypes analyzed with a sensitivity ranging between 10 and 100 copies of viral genome.

### Example 6

### Validation.

The multiplex BPV genotyping assay has been tested using DNA isolated from 42 warts from a total of 42 cattle from the same cowshed. The assay identified BPV types 1, 2, 5, 6, 8, 9 and 10 in mostly multiple infections. Using extracted DNA from hairs from cattle from another cowshed, BPV types 2, 3, 5, 6, 8 and 10 were found. This data indicated that the distribution of BPV differs in different cowsheds, and that multiple infections are highly prevalent.

## Claims

1. A composition comprising oligonucleotides selected from at least two different groups of oligonucleotides, said groups comprising at least one pair of oligonucleotides being capable of specifically amplifying polynucleotides comprised by a Bovine Papillomavirus (BPV), said BPV being selected from the group consisting of BPV-1, BPV-2, BPV-3, BPV-4, BPV-5, BPV-6, BPV-7, BPV-8, BPV-9, BPV-10, and BAPV-11.

2. The composition of claim 1, wherein said at least two groups of oligonucleotides are selected from the group consisting of
a) a group of oligonucleotides comprising
i) a pair of oligonucleotides having a nucleic acid sequence as shown in SEQ ID NO: 1 and SEQ ID NO: 2 or
ii) at least one pair of oligonucleotides which are capable of specifically amplifying polynucleotides which are specifically amplified by the oligonucleotides i); and
b) a group of oligonucleotides comprising
i) a pair of oligonucleotides having a nucleic acid sequence as shown in SEQ ID NO: 3 and SEQ ID NO: 4 or
ii) at least one pair of oligonucleotides which are capable of specifically amplifying polynucleotides which are specifically amplified by the oligonucleotides i); and
c) a group of oligonucleotides comprising
i) a pair of oligonucleotides having a nucleic acid sequence as shown in SEQ ID NO: 5 and SEQ ID NO: 6 or
ii) at least one pair of oligonucleotides which are capable of specifically amplifying polynucleotides which are specifically amplified by the oligonucleotides i); and
d) a group of oligonucleotides comprising
i) a pair of oligonucleotides having a nucleic acid sequence as shown in SEQ ID NO: 7 and SEQ ID NO: 6 or
ii) at least one pair of oligonucleotides which are capable of specifically amplifying polynucleotides which are specifically amplified by the oligonucleotides i); and
e) a group of oligonucleotides comprising
i) a pair of oligonucleotides having a nucleic acid sequence as shown in SEQ ID NO: 8 and SEQ ID NO: 9 or
ii) at least one pair of oligonucleotides which are capable of specifically amplifying polynucleotides which are specifically amplified by the oligonucleotides i); and
f) a group of oligonucleotides comprising
i) a pair of oligonucleotides having a nucleic acid sequence as shown in SEQ ID NO: 10 and SEQ ID NO: 11 or
ii) at least one pair of oligonucleotides which are capable of specifically amplifying polynucleotides which are specifically amplified by the oligonucleotides i); and
g) a group of oligonucleotides comprising
i) a pair of oligonucleotides having a nucleic acid sequence as shown in SEQ ID NO: 12 and SEQ ID NO: 13 or
ii) at least one pair of oligonucleotides which are capable of specifically amplifying polynucleotides which are specifically amplified by the oligonucleotides i); and
h) a group of oligonucleotides comprising
i) a pair of oligonucleotides having a nucleic acid sequence as shown in SEQ ID NO: 14 and SEQ ID NO: 15 or
ii) at least one pair of oligonucleotides which are capable of specifically amplifying polynucleotides which are specifically amplified by the oligonucleotides i); and
i) a group of oligonucleotides comprising
i) a pair of oligonucleotides having a nucleic acid sequence as shown in SEQ ID NO: 16 and SEQ ID NO: 17 or
ii) at least one pair of oligonucleotides which are capable of specifically amplifying polynucleotides which are specifically amplified by the oligonucleotides i); and
j) a group of oligonucleotides comprising
i) a pair of oligonucleotides having a nucleic acid sequence as shown in SEQ ID NO: 18 and SEQ ID NO: 19 or
ii) at least one pair of oligonucleotides which are capable of specifically amplifying polynucleotides which are specifically amplified by the oligonucleotides i).

3. The composition of any one of claims 1 or 2, wherein said oligonucleotides are selected from at least three, four, five, six, seven, eight, nine, ten groups of oligonucleotides.

4. A composition comprising at least two, three, four, five, six, seven, eight, nine, ten probe oligonucleotides, said oligonucleotides being capable of specifically hybridizing to polynucleotides comprised by a BPV, said BPV being selected from the group consisting of BPV-1, BPV-2, BPV-3, BPV-4, BPV-5, BPV-6, BPV-7, BPV-8, BPV-9, BPV-10, and BAPV-11.

5. The composition of claim 4, wherein said probe oligonucleotides are capable of specifically hybridizing to polynucleotides which are amplified by the oligonucleotides of claim 2.

6. The composition of any one of claims 4 or 5, wherein said probe oligonucleotides have a nucleic acid sequence as shown in SEQ ID NOs: 21 to 30 or wherein said oligonucleotides are capable to specifically hybridize to the antisense strand of oligonucleotides that have nucleic acid sequences as shown in SEQ ID NOs: 20 to 31.

7. A method for the simultaneous detection and / or identification of BPV types in a sample comprising the steps of
a) contacting a sample with a composition of any one of claims 1 to 3 under conditions which allow for the amplification of polynucleotides and
b) determining the presence and / or identity of BPV in said sample based on the amplified polynucleotides obtained in step a).

8. The method of claim 7, wherein the amplified polynucleotides are obtained by polymerase chain reaction.

9. The method of any one of claims 7 or 8, wherein the determination of the various amplified polynucleotides comprises the step of hybridizing the amplified polynucleotides with probe oligonucleotides of any one of claims 4 to 6.

10. Use of a composition of any one of claims 1 to 3 and / or any one of claims 4 to 6 for detecting and / or identifying BPV in a sample.

11. The use of claim 10, wherein different BPVs are detected and / or identified simultaneously.

12. A kit, preferably adopted for carrying out the method of any one of claims 7 to 11, comprising the composition of any one of claims 1 to 3 and / or any one of claims 4 to 6 and an instruction manual.
